# EUROPÄISCHE PATENTSCHRIFT

(11) **EP 1 197 200 B1**
(45) Veröffentlichungstag und Bekanntmachung des Hinweises auf die Patenterteilung: **07.06.2006**
(21) Anmeldenummer: 01123312.9
(22) Anmeldetag: 08.10.2001
(51) Int. Cl.: A61K 8/29, A61Q 5/00, A61Q 17/04

(54) **Haarpflegemittel**
Hair care product
Produit pour le soin des cheveux

(30) Priorität: 13.10.2000 DE 10050782
(43) Veröffentlichungstag der Anmeldung: 17.04.2002
(73) Patentinhaber: KPSS-Kao Professional Salon Services GmbH, 64297 Darmstadt (DE)
(72) Erfinder: Fath, Bettina, 69469 Weinheim (DE); Dubowoj, Polina, Aree Place Condominium Room 801, Klongton, Klongtoey, Bangkok 10110 (TH)

(56) Entgegenhaltungen:
- EP-A- 0 692 247
- EP-A- 0 898 955
- WO-A-00/40207
- WO-A-99/51193

## Beschreibung

Die vorliegende Erfindung betrifft ein Mittel zum Behandeln von menschlichem Haar, das diesem verbesserte Eigenschaften, insbesondere Naß- und Trockenkämmbarkeit, erhöhten Glanz, einen vollen und gleichzeitig lockeren Griff sowie guten Halt verleiht und gleichzeitig eine verbesserte Schutzwirkung vor allem gegen Umwelteinflüsse ausübt.

Mittel zum Konditionieren von menschlichen Haaren sind seit langem bekannt.
Sie enthalten in der Regel quaternäre Ammoniumverbindungen, die mindestens eine langkettige Alkyl- oder Alkenylgruppe aufweisen, und gegebenenfalls auch Polymere.
Solche Mittel werden üblicherweise als wäßrige Dispersionen bzw. Emulsionen, Mikroemulsionen, Gele oder auch in Aerosolform konfektioniert und als Haarspülungen, Kuren etc. eingesetzt.
Eine Übersicht über die bekannten Haarnachbehandlungsmittel und ihre Zusammensetzung findet sich in der Monographie von K. Schrader, Grundtagen und Rezepturen der Kosmetika, 2. Aufl. (1989), S. 722 bis 781, insbesondere S. 728 bis 737.
Diese bekannten Zusammensetzungen sind aber noch verbesserungsfähig.

Es wurde nunmehr gefunden, daß durch die Anwendung eines Haarbehandlungsmittels dem Haar verbesserte Eigenschaften, insbesondere ein deutlich erhöhter Volumeneffekt, leichtere Naß- und Trockenkämmbarkeit sowie einen dezenter Glanz, ein voller lockerer Griff und guter Halt sowie Schutz gegen negative Umwelteinflüsse verliehen wird, wenn dieses Mittel auf wäßriger Basis mindestens ein Glimmer/Titandioxid-Pigment mit einem Teilchendurchmesser von mindestens 90% im Bereich von 10 bis 250 Mikron, Grünen Tee Extrakt, und mindestens eine wasserlösliche oder öltösliche UV-absorbierende Substanz enthält.

Dieses Mittel wird, vorzugsweise nach einer Haarwäsche, gegebenenfalls im Anschluß an eine zuvor durchgeführte Haarfärbung oder Dauerwellung, auf das Haar aufgebracht, vorzugsweise in dieses einmassiert und nach der Einwirkung nicht entfernt.

Es handelt sich also um sogenannte "Leave on"-Produkte, im Gegensatz zu sogenannten "Rinse off"-Produkten, die nach einer kurzen Einwirkungszeit wieder aus dem Haar ausgespült werden.

Geeignete Glimmer-Titandioxid-Pigmente, die in den erfindungsgemäß zur Haarbehandlung verwendeten Mitteln vorzugsweise in einer Menge von 0,05 bis 10, insbesondere 0,1 bis 5 Gew.-%, bezogen auf die Gesamtzusammensetzung des Mittels, eingesetzt werden, sind an sich bekannt, beispielsweise unter der Bezeichnung "Timiron® ".
90% der Teilchen weisen dabei einen Teilchendurchmesser im Bereich von 10 bis 250, 80% vorzugsweise zwischen 20 und 150 Mikron auf.

Vorzugsweise wird die UV-absorbierende Substanz aus mindestens einer der folgenden Verbindungen ausgewählt:
4-Aminobenzoesäure und deren Estern und Salzen, 2-Phenylbenzimidazol-5-sulfonsäure und deren Alkali- und Aminsalzen, 4-Dimethylaminobenzoesäure und deren Estern und Salzen, Zimtsäure und deren Estern und Salzen, 4-Methoxyzimtsäure und deren Estern und Salzen, Salicylsäure und deren Estern und Salzen, 2,4-Dihydroxybenzophenon, 2,2',4,4'-Tetrahydroxybenzophenon, 2-Hydroxy-4-methoxybenzophenon und dessen 5-Sulfonsäure bzw. deren Natriumsalz, 2,2'-Dihydroxy-4,4'-dimethoxybenzophenon, 2-Hydroxy-5-chlorbenzophenon, 2,2'-Dihydroxy-4-methoxybenzophenon, 2,2'-Dihydroxy-4,4'-dimethoxy-5,5'-disulfobenzophenon bzw. dessen Natriumsalzen, 2-Hydroxy-4-octyloxybenzophenon, 2-Hydroxy-4-methoxy-4'-methylbenzophenon, 3-Benzylidencampher, 3-(4'-Sulfo)-benzylidenbornan-2-on und dessen Salzen und/oder 3-(4'-Methylbenzyliden)-DL-campher. Der bevorzugte Anteil des UV-Absorbers beträgt 0,05 bis 2,5, vorzugsweise 0,1 bis 1 Gew.-%, berechnet auf die Gesamtzusammensetzung des Mittels.

Die erfindungsgemäßen Mittel enthalten zusätzliche weitere haarkonditionierende Wirkstoffe.
Geeignete haarkonditionierende Wirkstoffe, deren Menge in den erfindungsgemäß verwendeten Mitteln üblicherweise bei 0,25 bis 15, insbesondere 0,5 bis 10, vorzugsweise 0,75 bis 7,5 Gew.-%, bezogen auf die Gesamtmenge des Mittels, sind beispielsweise kationische Tenside, vor allem quaternäre Ammoniumverbindungen.

Geeignete langkettige quaternäre Ammoniumverbindungen, die allein oder im Gemisch miteinander eingesetzt werden können, sind beispielsweise Cetyltrimethylammoniumchlorid, Dimethyldicetylammoniumchlorid, Trimethylcetylammoniumbromid, Dimethyldistearylammoniumchlorid, Stearyltrimethylammoniumchlorid, Dimethylstearylbenzylammoniumchlorid, Benzyltetradecylodimethylammoniumchlorid, Dimethyl- dihydriertes-Talgammoniumchlorid, Laurylpyridiniumchlorid, Lauryldimethylbenzylammoniumchlorid, Behenyltrimethylammoniumchlorid, Lauryltrimethylammoniumchlorid, Tris-(oligooxyethyl)alkylammoniumphosphat, Cetylpyridiniumchlorid, etc.

Gut geeignet sind auch die in der EP-A 472 107 geoffenbarten quaternären Ammoniumsalze.
Im Prinzip sind alle quaternären Ammoniumverbindungen, wie sie im jeweils gültigen "CTFA International Cosmetic Ingredient Dictionary" unter dem Trivialnamen "Quaternium" aufgeführt sind, einsetzbar.

Ihr Anteil liegt vorzugsweise bei 0,5 bis 10, insbesondere 1 bis 7,5 Gew.-%, berechnet auf die Gesamtzusammensetzung.

Besonders geeignete langkettige quaternäre Ammoniumverbindungen sind Esterquats der allgemeinen Formel (I) 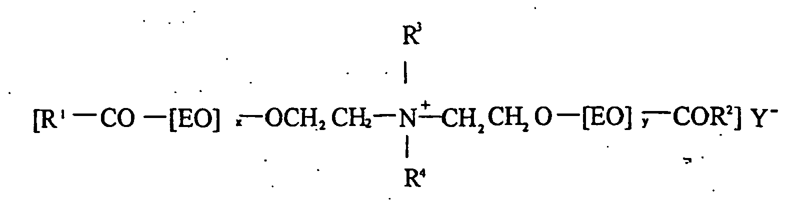 in der R¹ und R² für eine gegebenenfalls hydroxysubstituierte C₈-C₂₂-Alkyl- oder Alkenylgruppe, R³ und R⁴ für eine C₁-C₃-Alkylgruppe oder eine Gruppe -CH₂-CH₂-O-[EO]_{z}H sowie x,y und z für 0 bis 5 und Y⁻ für ein Anion stehen.

Ein besonders bevorzugtes Esterquat ist im Rahmen der Erfindung eine solche, in der die Reste R¹ und R² jeweils eine Oleylgruppe oder eine C₁₂-C₁₈-Alkylgruppe, der Rest R³ eine Methylgruppe und der Rest R⁴ eine Gruppe -CH₂-CH₂-O-[E0]_{z}-H bedeuten.

Das Anion Y⁻ ist vorzugsweise ein Halogenid wie Cl⁻ oder Br⁻, ein niederes Alkylsulfat, z.B. Methosulfat und Ethosulfat, oder ein Alkylphosphat, jedoch können selbstverständlich auch andere Reste eingesetzt werden.

Diese Verbindungen sind an sich bekannt und beispielsweise unter den Handelsnamen "Schercoquat^{A}", "Dehyquart^{R}F30" und "Tetranyl^{R}" im Handel.

Der Einsatz dieser Verbindungen, in Haarpflegemitteln ist ebenfalls bereits bekannt und beispielsweise in der WO-A 93/10748, der WO-A 92/06899 und der WO-A 94/16677 beschrieben, wo sich jedoch keinerlei Hinweise auf die erfindungsgemäße Kombination und deren vorteilhafte Eigenschaften finden.

Ebenso geeignet sind "Amidoquats" der allgemeinen Formel 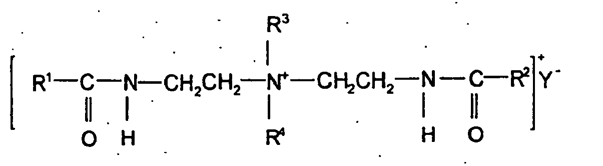 in der R¹ und R² jeweils für eine gegebenenfalls hydroxysubstituierte C₈-C₂₂-Alkyl- oder Alkenylgruppe, R³ und R⁴ für eine C₁-C₃-Alkylgruppe oder eine Gruppe -CH₂₋CH₂-O-[EO]ₓ-H, sowie x für 0 bis 5, Y⁻ für ein Anion stehen.

Bevorzugte Reste R¹ und R² sind C₁₂-C₁₈-Alkyl- und Oleylreste, der Rest R³ eine Methylgruppe und der Rest R⁴ eine Gruppe -CH₂-CH₂-O[EO]ₓ-H, worin x 0 bis 3 ist; Y⁻ ist vorzugsweise ein Methosulfat-, Ethylsulfat-, Chlorid oder Phosphatanion.

Diese Verbindungen sind an sich bekannt und beispielsweise unter den Markenbezeichnungen "INCROQUAT® HO" oder "OCS" im Handel.

Weitere geeignete haarkonditionierende Wirkstoffe sind synthetische oder natürliche haarkonditionierende Polymere, vorzugsweise in einer Menge von 0,1 bis 10, insbesondere 0,25 bis 5 Gew.-% der Gesamtzusammensetzung.

Besonders bevorzugt sind hierbei die unter der CTFA-Bezeichnung "Polyquaternium" bekannten kationischen (Co-)Polymeren, jedoch können auch nichtionische, anionische und/oder amphotere Polymere, beispielsweise solche vom Typ "Amphomer^{R}", alleine oder im Gemisch verwendet werden.

Weitere haarkonditionierende Wirkstoffe sind lipophile, d.h. fett- und ölhaltige Substanzen, zu denen auch Wachse zählen, sind insbesondere natürliche Öle wie Avocadoöl, Cocosöl, Palmöl, Sesamöl, Erdnußöl, Spermöl, Sonnenblumenöl, Mandelöl, Pfirsichkernöl, Weizenkeimöl, Macadamianußöl, Nachtkerzenöl, Jojobaöl, Ricinusöl, oder auch Oliven- bzw. Sojaöl, Lanolin und dessen Derivate, ebenso Mineralöle wie Paraffinöl und Vaseline.

Synthetische Öle und Wachse sind beispielsweise Silikonöle, Polyethylengykole, etc. Weitere geeignete hydrophobe Komponenten sind insbesondere Fettalkohole, vorzugsweise solche mit etwa 8 bis 22 Kohlenstoffatomen im Molekül wie Myristyl-, Cetyl-, Stearylalkohol, Wachsalkohole und Fettsäureester wie Isopropylmyristat, -palmitat, -stearat und -isostearat, Oleyloleat, Isocetylstearat, Hexyllaurat, Dibutyladipat, Dioctyladipat, Myristylmyristat, Oleylerucat, Polyethylenglykol- und Polyglycerylfettsäureester wie PEG-7-glycerylcocoat, Cetylpalmitat, etc.

Diese hydrophoben Komponenten sind in den erfindungsgemäß verwendeten Zusammensetzungen vorzugsweise in einer Gesamtmenge von 0,5 bis 15, insbesondere 1 bis 10, vor allem 1,5 bis 7,5 Gew.-%, berechnet auf die Gesamtzusammensetzung, enthalten.

Ein weiterer Bestandteil der erfindungsgemäßen Zusammensetzung ist Grüner Tee-Extrakt.
Dieser Tee-Extrakt wird aus Blättern, Blattknospen und zarten Stielen des Teestrauchs, Camellia sinensis bzw. Camellia oleifera, durch wäßrige bzw. wäßrigalkoholische Extraktion und anschließende Sprühtrocknung erhalten.

Bei grünem Tee handelt es sich um die aus den Arten Thea sinensis bzw. Thea assamica gewonnenen, im Gegensatz zum schwarzen Tee nicht fermentierten Produkte.

Eine Übersicht über die biologische und pharmakologische Wirkung grünen Tees sowie seine Inhaltsstoffe findet sich z.B. in einem Artikel von A. Pistorius, SÖFW-Journal, 122. Jahrgang, No. 7/1996, S. 468-471, auf den Bezug genommen wird.

Der Gehalt an Extrakt aus grünem Tee in den erfindungsgemäßen Zusammensetzungen ist variabel. Er liegt vorzugsweise bei 0,01 bis 10, vorzugsweise 0,05 bis 5, insbesondere 0,25 bis 2,5 Gew.-%, berechnet auf die Gesamtzusammensetzung des Mittels und den pulverförmigen Extrakt.

Weitere geeignete haarkonditionierende Zusatzstoffe sind Ceramide der allgemeinen Formel 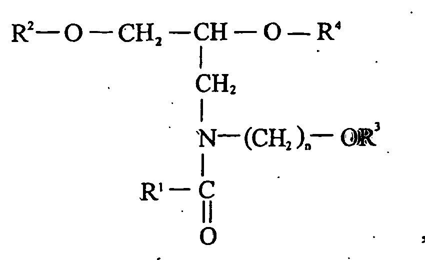 worin R¹ und R² gleiche oder verschiedene Alkyl- bzw. Alkenylreste mit 10 bis 22 Kohlenstoffatomen bedeuten, R³ für Wasserstoff oder eine Methyl-, Ethyl-, n-Propyl- oder Isopropylgruppe steht, R⁴ Wasserstoff, eine Hydroxymethyl-,Hydroxyethyl-, Dihydroxyethyl- oder Dihydroxypropylgruppe, und n eine ganze Zahl von 1 bis 6 bedeuten, insbesondere der Art, wie es aus der EP 227 994 A1 und der WO-A 96/37462 bekannt ist, jedoch sind auch andere Ceramide, beispielsweise die aus der WO-A 97/15724 oder der EP 647 617 B1 bekannten Ceramide, geeignet.

Die bevorzugten Gruppen R¹ und R² sind C₁₂-C₁₈-Alkylreste; n ist eine Zahl von 1 bis 3, R³ bedeutet vorzugsweise Wasserstoff oder einen Methylrest, und R⁴ Wasserstoff oder einen Dihydroxypropylrest.
Besonders bevorzugt sind Verbindungen, in denen R¹ einen C₁₂-C₂₄-Alkylrest, insbesondere eine C₁₃H₂₇-Alkylgruppe, R² einen C₁₄C₁₈-Alkylrest, insbesondere eine C₁₆H₃₃-Alkylgruppe, R³ einen Methylrest, R⁴ eine 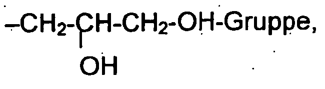 und n 3 darstellen, oder eine Verbindung, wo R¹ für einen C₁₅-C₃₁-Alkylrest, R² für einen C₁₆H₃₃-Alkylrest, R³ und R⁴ für je ein Wasserstoffatom und n für 2 stehen.

Deren Menge in den erfindungsgemäß eingesetzten Haarbehandlungsmitteln liegt zweckmäßigerweise bei 0,01 bis 10, vorzugsweise 0,05 bis 7,5, insbesondere 0,1 bis 5 Gew.-%, berechnet auf die Gesamtzusammensetzung.

Weitere geeignete haarkonditionierende Wirkstoffe sind wasserunlösliche Vitamine und deren Derivate, beispielsweise Vitamin E und dessen Ester wie Tocopherolacetat, -propionat, -palmitat, etc.

Weitere Zusatzstoffe, deren Art und Menge natürlich von der Applikationsform des Mittels abhängig sind, sind Fette, Fettalkohole, Emulgatoren, pH-Regulatoren, Lösungs- und Verdünnungsmittel, Lösungsvermittler, Konservierungsmittel, Parfums, etc.

Die erfindungsgemäßen Mittel können auch Tenside enthalten.
Als Tenside sind insbesondere nichtionische Tenside geeignet.
Besonders bevorzugte nichtionische Tenside sind die bekannten C₈-C₁₈₋Alkylpolyglucoside, insbesondere mit einem Polykondensationsgrad von 1,2 bis 3, vor allem solche der allgemeinen Formel

R-O-(R¹O)ₙ-Zₓ,

worin R eine Alkylgruppe mit 8 bis 18 Kohlenstoffatomen, R¹ eine Ethylen- oder Propylengruppe, Z. einen Saccharidrest mit 5 bis 6 Kohlenstoffatomen, n eine Zahl von 0 bis 10 und x eine Zahl zwischen 1 und 2,5 bedeuten, in einer Menge von 1 bis 10, insbesondere 2,5 bis 7,5 Gew.-% der Gesamtzusammensetzung.

Andere geeignete nichtionische Tenside sind beispielsweise die verschiedenen Sorbitanester, wie Polyethylenglykolsorbitanstearinsäureester, Fettsäurepolyglykolester oder auch Mischkondensate aus Ethylenoxid und Propylenoxid, wie sie beispielsweise unter der Handelsbezeichnung "Pluronics" im Verkehr sind.

Besonders geeignete Tenside sind amphotere und/oder zwitterionische oberflächenaktive Substanzen in einer Menge von 0,5 bis 15, vorzugsweise von 1 bis 5 Gew.-%, bezogen auf die Gesamtzusammensetzung.

Als solche sind insbesondere die verschiedenen bekannten Betaine wie Fettsäureamidoalkylbetaine und Sulfobetaine, beispielsweise Laurylhydroxysulfobetain, zu nennen; auch langkettige Alkylaminosäuren wie Cocoaminoacetat, Cocoaminopropionat und Natriumcocoamphopropionat und -acetat haben sich als geeignet erwiesen.
Im einzelnen können Betaine der Struktur 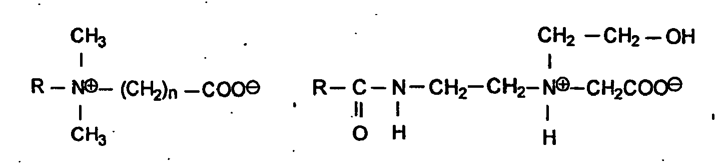 wobei R eine C₈-C₁₈-Alkylgruppe und n 1 bis 3 bedeuten,
Sulfobetaine der Struktur

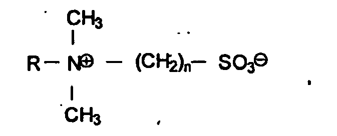

Alkylamidobetaine der allgemeinen Formel 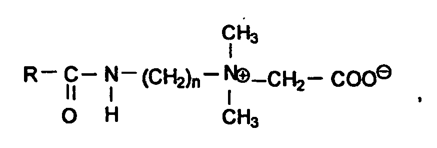 wobei R eine C₈-C₁₈-Alkylgruppe und n 1 bis 3 bedeuten, verwendet werden. Durch den Zusatz dieser Substanzen werden insbesondere die haarschützenden und -konditionierenden Eigenschaften des Produkts weiter verbessert.

Zusätzlich zu oder anstelle der genannten amphoteren bzw. zwitterionischen Tenside können die Haarpflegemittel mindestens ein C₁₂-C₁₈-Alkylamidopropyldimethyl- bzw.-diethylamin enthalten, beispielsweise Stearyl-, Oleyl- oder Cocoamidopropyldimethylamin.

Dessen Anteil liegt bei 0,1 bis 10, vorzugsweise 0,25 bis 5 Gew.-% der Gesamtzusammensetzung.

Daneben sind natürlich auch anionische und/oder andere amphotere bzw. zwitterionische Tenside geeignet.

Ein weiterer bevorzugter Bestandteil in den erfindungsgemäßen Mitteln sind Pflanzenextrakte in einer Menge von 0,01 bis 10, vorzugsweise 0,1 bis 7,5, insbesondere 0,5 bis 5 Gew.-%, berechnet als Trockenrückstand desselben auf die Gesamtmenge des Mittels.

Geeignete, an sich bekannte wäßrige (z.B Wasserdampf-destillierte), alkoholische oder wäßrig-alkoholische Pflanzenextrakte sind insbesondere Extrakte von Blättern, Früchten, Blüten, Wurzeln, Rinden oder Stämmen von Aloe, Ananas, Artischoken, Arnika, Avocado, Baldrian, Bambus, Bilsenkraut, Birke, Brennesseln, Echinacea, Efeu, Engelwurz, Enzian, Farnen, Fichtennadeln, Gänsefingerkraut, Ginseng, Ginster, Hafer, Hagebutten, Hamamelis, Heublumen, Holunder, Hopfen, Huflattich, Johannisbeeren, Kamillen, Karotten, Kastanien, Klee, Klettenwurzeln, Kokosnuß, Korn, Lindenblüten, Maiglöckchen, Meeralgen, Melisse, Mistel, Passionsblumen, Ratanhia, Ringelblumen, Rosmarin, Roßkastanien, Rotdorn, Salbei, Schachtelhalm, Scharfgarbe, Schlüsselblumen, Taubnesseln, Thymian, Walnüssen, Weinblättern, Weißdorn, etc.

Geeignete Handelsprodukte sind beispielsweise die verschiedenen "Extrapone^{R}", "Herbasol^{R}", "Sedaplant^{R}" und "Hexaplant^{R}". Extrakte und deren Herstellung sind auch in "Hagers Handbuch der pharmazeutischen Praxis", 4. Aufl., beschrieben.

Die erfindungsgemäß verwendeten haarkonditionierenden Mittel liegen vorzugsweise als, wäßrige oder wäßrig/alkoholische Lösung, wäßrige Emulsion, Mikroemulsion, Dispersion oder opakes oder transparentes Gel vor, und können auch als Aerosole konfektioniert werden. Solche Zusammensetzungen und ihre Herstellung sind dem Fachmann grundsätzlich bekannt und bedürfen daher keiner näheren Erläuterung.

Der pH-Wert der erfindungsgemäß verwendeten Haarbehandlungsmittel ist nicht kritisch; er kann vorzugsweise bei 3 bis 8, insbesondere zwischen 4 und 6,5, liegen.

Ebenso liegt die Viskosität im allgemein Bereich und ist selbstverständlich abhängig von der Darreichungsform des Mittels.

Die folgenden Beispiele beschreiben die Erfindung im Detail:

### Beispiel 1

Es wurde eine **"Leave-on Kur"** folgender Zusammensetzung hergestellt:

| | |
|---|---|
| Cetylstearylalkohol | 2,5 (Gew.-%) |
| Isopropylmyristat | 0,2 |
| Stearamidopropyldimethylamin | 0,6 |
| 2-Phenylbenzimidazol-5-sulfonsäure | 0,3 |
| Milchsäure | 0,3 |
| Parfum | 0,1 |
| Grüner Tee-Extrakt | 0,5 |
| Konservierungsmittel | 0,3 |
| Mica/TiO₂-Pigment (Timiron^{R} Diamond Cluster; | 0,5 |
| mittl. Teilchendurchmesser: | |
| 90% zwischen 20 und 150 µm) | |
| pH-Wert: | ~4,5 |
| Wasser | ad 100,0 |

An leicht strapazierten Haarsträhnen wurde folgender Blindversuch durchgeführt:

In 5 Strähnen wurde nach der Haarwäsche mit einem handelsüblichen Shampoo in das noch nasse Haar eine Zusammensetzung 1 entsprechend dem obigen Beispiel einmassiert.

Weitere 5 Strähnen wurden mit einer mit der Zusammensetzung 1 identischen Zusammensetzung 1A, die jedoch keine Pigmentteilchen enthielt, in identischer Weise behandelt.

Die Haarsträhnen wurden vor und nach dem Trocknen von jeweils 2 Friseuren nach der Präferenzmethode beurteilt.

| **Ergebnis** | **Präferenz** | |
|---|---|---|
| | **Zusammensetzung 1** | **Zusammensetzung 1A** |
| **a) Nasses Haar** | | |
| Haargefühl | 4 (Seidig weich, glatt) | 1 (Weich, glitschig) |
| Naßkämmbarkeit | 5 (Sehr gut) | 0 (Gut) |

| **b) Trockenes Haar** | | |
|---|---|---|
| Volumen, Fülle | 4 | 1 |
| Halt | 4 | 1 |
| Glanz | 4 | 1 |
| Trockenkämmbarkeit | 5 (Sehr gut) | 0 (Gut) |
| Griff | 5 (Seidig weich, glatt vom Ansatz bis zur Spitze) | 0 (Fester, Spitzen rauher) |

Dieses Ergebnis zeigt die überraschende Überlegenheit des erfindungsgemäßen Mittels.

Diese Unterschiede traten noch verstärkt nach 24-stündiger UV-Bestrahlung der behandelten Strähnen auf.

### Beispiel 2 (nicht erfindungsgemäß)

| | |
|---|---|
| 1,2-Propylenglykol | 2,00 (Gew.-%) |
| CarbopoI^{R}ETD 2020 (Acrylates/ | 0,50 |
| C₁₀-C₂₀-Alkyl acrylate crosspolymer) | |
| Cocoamidopropylbetain | 1,00 |
| Ethanol | 17,50 |
| PPG 9 | 0,70 |
| PEG-60 Hydriertes Ricinusöl | 0,50 |
| NaOH (32%) | 0,25 |
| Parfum | 0,10 |
| PEG-25 PABA | 0,80 |
| Mandelöl | 2,00 |
| Mica/TiO₂-Pigment (Timiron^{R} Diamond Cluster; | 0,60 |
| 90 % mit durchschnittl. Teilchendurchmesser | |
| von 20 bis 150µm) | |
| Wasser | ad 100,0 |
| pH-Wert: | ~6,0 |

Mit diesem Produkt wurde eine ähnliche Verbesserung der Haareigenschaften wie mit demjenigen nach Beispiel 1 erzielt.

### Beispiel 3

Ein **Styting-Gel** der folgenden Zusammensetzung:

| | |
|---|---|
| Tocopherylacetat | 0,5 (Gew.%) |
| Mica/TiO₂-Pigment (Timiron^{R} Gleamer Flake; | 0,5 |
| mittl. Teilchengröße: | |
| 90% zwischen 20 und 100µm) | |
| Carbopol^{R} ETD 2001 (Carbomer) | 0,5 |
| Ethanol | 11,0 |
| VinylpyrrolidonVinylacetat-Copolymer | 5,5 |
| (Luviskol^{R} VA 551) | |
| Aminomethylpropanol | 0,4 |
| Cocoamidopropylbetain | 1,0 |
| PEG-60 Hydriertes Ricinusöl | 0,4 |
| Grüner Tee-Extrakt | 0,4 |
| Parfum | 0,3 |
| Benzophenone-4 | 0,3 |
| Konservierungsmittel | 0,3 |
| pH-Wert: | ~6,8 |
| Wasser | ad 100,0 |

ergab nach dem Einmassieren in frisch gewaschenes Haar eine exzellente haarpflegende Wirksamkeit sowie einen hervorragenden Lichtschutz des Haares.

### Beispiel 4

Eine **Leave-on Kur** der Zusammensetzung

| | |
|---|---|
| Cetearylalkohol | 3,00 |
| Isopropylpalmitat | 0,20 |
| Sphingolipid E | 0,10 |
| Quaternium-80 | 0,10 |
| Mica/TiO₂-Pigment (Timiron^{R} Diamond Chuster; | 0,15 |
| mittl. Teilchengröße:20 und 150µm) | |
| Grüner Tee-Extrakt | 0,05 |
| Benzophenone-4 | 0,10 |
| Cocoamidopropyldimethylamin | 0,60 |
| Milchsäure | 0,35 |
| Konservierungsmittel | 0,45 |
| Parfumöl | 0,20 |
| Ceteareth-20 | 0,40 |
| Wasser | ad 100,00 |

wurde nach der Zweiseitenmethode auf eine Hälfte gewaschenen Haares von 8 Probandinnen aufgebracht.
Eine identische Zusammensetzung (4A), bei der jedoch Cocoamidopropyldimethylamin und Benzophenone-4 durch Wasser ersetzt waren, wurde in die andere Hälfte des Haares einmassiert.
Nach 10-minütiger Einwirkung wurden die Haarhälften durch zwei erfahrene Friseure begutachtet, wobei weder die Friseure noch die Probandinnen die Zuordnung der Produkte zu den jeweiligen Haarhälften kannten (Doppelblind-Methode).

Es wurde folgendes Ergebnis erzielt:

| **Eigenschaften** | **Zusammensetzung 4** | | **Zusammensetzung 4 A** |
|---|---|---|---|
| | **Besser** | **Gleich** | **Besser** |
| **Nasses Haar** | | | |
| Kämmbarkeit | 5 | 2 | 1 |
| Griff, Lockerheit | 6 | 1 | 1 |

| **Trockenes Haar** | | | |
|---|---|---|---|
| Kämmbarkeit | 6 | 1 | 1 |
| Griff, Lockerheit | 6 | 1 | 1 |
| Volumen | 5 | 2 | 1 |
| Glanz | 5 | 2 | 1 |

## Patentansprüche

1. Haarpflegemittel auf wäßriger Basis, enthaltend
a) mindestens eine wasserlösliche oder öllösliche UV-absorbierende Substanz,
b) mindestens ein Glimmer/Titaniumdioxid-Pigment, wobei mindestens 90 Gew.-% desselben eine Teilchengröße zwischen und 10 und 250 Mikron aufweisen, und
c) Grünen Tee Extrakt.

2. Haarpflegemittel nach Anspruch 1, **dadurch gekennzeichnet, daß** das Pigment zu 80 bis 90 Gew.-% aus Glimmer und zu 10 bis 20 Gew.-% aus Titandioxid besteht.

3. Haarpflegemittel nach Anspruch 1 oder 2, **dadurch gekennzeichnet, daß** der Anteil des Glimmer/Titandioxid-Pigments 0,05 bis 5 Gew.-%, berechnet auf die Gesamtzusammensetzung, beträgt.

4. Haarpflegemittel nach einem öder mehreren der Ansprüche 1 bis 3, **dadurch gekennzeichnet, daß** der UV-Absorber ausgewählt ist aus mindestens einer der Verbindungen 4-Aminobenzoesäure und deren Estern und Salzen, 2-Phenylbenzimidazol-5-sulfonsäure und deren Alkali- und Aminsalzen, 4-Dimethylaminobenzoesäure und deren Estern und Salzen, Zimtsäure und deren Estern und Salzen, 4-Methoxyzimtsäure und deren Estern und Salzen, Salicylsäure und deren Estern und Salzen, 2,4-Dihydroxybenzophenon, 2,2',4,4'-Tetrahydrobenzophenon, 2-Hydroxy-4-methoxybenzophenon und dessen 5-Sulfonsäure bzw. deren Natriumsalz, 2,2'-Dihydroxy-4,4'-dimethoxybenzophenon, 2-Hydroxy-5-chlorbenzophenon, 2,2'-Dihydroxy-4-methoxybenzophenon, 2-2'-Dihydroxy-4,4'-dimethoxy-5,5'-disulfobenzophenon bzw. dessen Natriumsalzen, 2-Hydroxy-4-octyloxybenzophenon, 2-Hydroxy-4-methoxy-4'-methylbenzophenon, 3-Benzylidencampher, 3-(4'-Sulfo)-benzylidenbornan-2-on und dessen Salzen und/oder 3-(4'-Methylbenzyliden)-DL-campher.

5. Haarpflegemittel nach einem oder mehreren der Ansprüche 1 bis 4, **dadurch gekennzeichnet, daß** der Anteil des UV-Absorbers 0,05 bis 2,5 Gew.-%, berechnet auf die Gesamtzusammensetzung, beträgt.

6. Haarpflegemittel nach einem oder mehreren der Ansprüche 1 bis 5, **dadurch gekennzeichnet, daß** es 0,5 bis 15 Gew.-% mindestens eines amphoteren bzw. zwitterionischen Tensids, berechnet auf die Gesamtzusammensetzung, enthält.

7. Haarpflegemittel nach Anspruch 6, **dadurch gekennzeichnet, daß** es 1 bis 5 Gew.-%, berechnet auf die Gesamtzusammensetzung, mindestens eines Betains enthält.

8. Haarpflegemittel nach einem oder mehreren der Ansprüche 1 bis 7, **dadurch gekennzeichnet, daß** es 0,1 bis 10 Gew.-% mindestens eines C₁₂-C₁₈₋Alkylamidopropyldimethyl- oder -diethylamins enthält.

## Claims

1. Hair care composition on aqueous basis, comprising
a) at least one water-soluble or oil-soluble UV-absorbing substance,
b) at least one mica /titanium dioxide pigment, whereby at least 90 % by weight thereof have a particle size between about 10 and 250 microns, and
c) green tea extract.

2. Hair care composition according to claim 1, wherein 80% to 90 % by weight of the pigment consist of mica, and 10 % to 20 % by weight thereof consist of titan dioxide.

3. Hair care composition according to claim 1 or 2, containing the mica/titanium dioxide pigment in an amount between 0.05 % to 5 % by weight, calculated to the total composition.

4. Hair care composition according to one or more of claims 1 to 3, wherein the UV-absorber is selected from at least one of the compounds 4-aminobenzoic acid and the esters and salts thereof, 2-phenyl benzimidazole-5-sulfonic acid and the alkali and amine salts thereof, 4-dimethyl aminobenzoic acid and the esters and salts thereof, cinnamic acid and the esters and salts thereof, 4-methoxy cinnamic acid and the esters and salts thereof, salicylic acid and the esters and salts thereof, 2.4-dihydroxybenzophenone, 2.2'.4.4'-tetrahydroxybenzophenone, 2-hydroxy-4-methoxybenzophenone and its 5-sulfonic acid or the sodium salt thereof, 2.2'-dihydroxy-4.4'-dimethoxybenzophenone, 2-hydroxy-5-chlorobenzophenone, 2.2'-dihydroxy-4-methoxybenzophenone, 2.2'-dihydroxy-4.4'-dimethoxy-5.5'-disulfobenzophenone or the sodium salt thereof, 2-hydroxy-4-octyloxybenzophenone, 2-hydroxy-4-methoxy-4'-methyl benzophenone, 3-benzylidene campher, 3-(4'-sulfo)-benzylidene bornane-2-one and the salts thereof and/or 3-(4'-methyl benzylidene)-DL-campher.

5. Hair care composition according to one or more of claims 1 to 4, wherein the proportion of the UV-absorber is from t 0.05 % to 2.5 % by weight, calculated to the total composition.

6. Hair care composition according to one or more of claims 1 to 5, **characterized by** containing 0.5 % to 15 % by weight of at least one amphoteric or zwitterionic surfactant, calculated to the total composition.

7. Hair care composition according to claim 6, **characterized by** containing 1 % to 5 % by weight, calculated to the total composition, of at least one betaine.

8. Hair care composition according to one or more of claims 1 to 7, **characterized by** containing 0.1 % to 10 % by weight of at least one C₁₂-C₁₈-alkyl amidopropyl dimethyl or diethyl amine.

## Revendications

1. Agent de soin des cheveux à base aqueuse, qui contient :
a) au moins une substance d'absorption des UV, soluble dans l'eau ou soluble dans les huiles,
b) au moins un pigment au mica et au dioxyde de titane, au moins 90 % en poids de ce pigment étant en particules d'une taille comprise entre 10 et 250 micromètres et
c) un extrait de thé vert.

2. Agent de soin des cheveux selon la revendication 1, **caractérisé en ce que** le pigment est constitué de 80 à 90 % en poids de mica et de 10 à 20 % en poids de dioxyde de titane.

3. Agent de soin des cheveux selon les revendications 1 ou 2, **caractérisé en ce que** sa teneur en pigment au mica et au dioxyde de titane est de 0,05 à 5 % en poids par rapport à la composition totale.

4. Agent de soin des cheveux selon l'une ou plusieurs des revendications 1 à 3, **caractérisé en ce que** l'agent d'absorption des UV est sélectionné parmi au moins l'un des composés acide 4-aminobenzoïque et ses esters et sels, acide 2-phénylbenzimidazole-5-sulfonique et ses sels de métal alcalin et d'amine, acide 4-diméthyaminobenzoïque et ses esters et sels, acide cinnamique et ses esters et sels, acide 4-méthoxycinnamique et ses esters et sels, acide salicylique et ses esters et sels, 2,4-dihydrobenzophénone, 2,2',4,4'-tétrahydrobenzophénone, 2-hydroxy-4-méthoxybenzophénone, son acide 5-sulfonique ainsi que le sel de sodium de ce dernier, 2,2'-dihydroxy-4,4'-diméthoxybenzophénone, 2-hydroxy-5-chlorobenzophénone, 2,2'-dihydroxy-4-méthoxybenzophénone, 2,2'-dihydroxy-4,4'-diméthoxy-5,5'-disulfobenzophénone et ses sels de sodium, 2-hydroxy-4-octyloxybenzophénone, 2-hydroxy-4-méthoxy-4'-méthylbenzophénone, 3-benzylidènecamphre, 3-(4'-sulfo)-benzylidènebornane-2-one et ses sels et/ou 3-(4'-méthylbenzylidène)-DL-camphre.

5. Agent de soin des cheveux selon l'une ou plusieurs des revendications 1 à 4, **caractérisé en ce que** par rapport à la composition totale, sa teneur en agent d'absorption des UV est de 0,05 à 2,5 % en poids.

6. Agent de soin des cheveux selon l'une ou plusieurs des revendications 1 à 5, **caractérisé en ce qu'**il contient de 0,5 à 15 % en poids d'au moins un agent tensioactif amphotère ou zwitterionique par rapport à la composition totale.

7. Agent de soin des cheveux selon la revendication 6, **caractérisé en ce que** par rapport à la composition totale, il contient de 1 à 5 % en poids d'au moins une bétaïne.

8. Agent de soin des cheveux selon l'une ou plusieurs des revendications 1 à 7, **caractérisé en ce qu'**il contient de 0,1 à 10 % en poids d'au moins une (alkyle en C₁₂ à C₁₈)amidopropyldiméthyl- ou diéthylamine.
